(19) Europäisches Patentamt
European Patent Office
Office européen des brevets

(11) Publication number: 0 123 495
B1

(12) # EUROPEAN PATENT SPECIFICATION

(45) Date of publication of the patent specification: 29.10.86

(51) Int. Cl.⁴: C 07 C 51/285, C 07 C 55/14, C 07 C 63/06

(21) Application number: 84302585.9

(22) Date of filing: 16.04.84

(54) **Process for preparing carboxylic acids from vicinal water-soluble diols.**

(30) Priority: 15.04.83 IT 2060583

(43) Date of publication of application: 31.10.84 Bulletin 84/44

(45) Publication of the grant of the patent: 29.10.86 Bulletin 86/44

(84) Designated Contracting States: BE CH DE FR GB LI NL

(56) References cited: US - A - 3 855 257

(73) Proprietor: Montedison S.p.A., 31, Foro Buonaparte, I-20121 Milan (IT)

(72) Inventor: Venturello, Carlo, 135, Via XXIII Marzo, Novara (IT)
Inventor: Ricci, Marco, 5, Via Brescia, Novara (IT)

(74) Representative: Whalley, Kevin et al, Marks & Clerk 57/60 Lincoln's Inn Fields, London WC2A 3LS (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



ACTORUM AG

## Description

The present invention relates to a process for preparing carboxylic acids from vicinal water-soluble diols (= dihydroxy compounds). More particularly, the invention relates to a process for the preparation of monocarboxylic and bicarboxylic acids by oxidative scission with $H_2O_2$ of vicinal diols soluble in water.

The monocarboxylic and bicarboxylic acids obtainable by oxidative scission of vicinal diols soluble in water are chemical products of considerable economic importance. Besides representing useful intermediates for organic syntheses, they have various industrial applications. Thus, for instance, adipic acid is used in the synthesis of polyamides, while benzoic acid is a useful intermediate for various syntheses; for instance, its esters are widely used as plasticizers.

The direct, selective oxidation of vicinal diols to carboxylic acids is of considerable interest because of the availability of these substrates that may be prepared starting from petrochemical products, for instance by hydroxylation of olefins.

The vicinal diols may be oxidized stoichiometrically to carboxylic acids by means of the use of a variety of oxidizers such as $KMnO_4$, cerium (IV) salts and $K_2Cr_2O_7$.

More conveniently there may be used catalytic processes based on the use of Caro acid (with vanadium based catalysts), of peracetic acid (with various catalysts such as $RuCl_3$ or Co, Mn, Ni, Fe or Pd acetates), or of sodium periodate (with $KMnO_4$ as a catalyst).

However, all the aforesaid processes suffer from serious disadvantages of an economic nature and/or connected with pollution problems. For instance, the use of sodium periodate as oxidant may on the one hand be economically convenient only if it were possible to recover it completely, an operation which however is rather difficult. On the other hand, the use of peracetic acid, which is relatively costly, presents the problem of the recovery of the acetic acid that is formed as a by-product of the reaction, and of its use.

It is also known that the vicinal diols are oxidized to carboxylic acids by molecular oxygen in the presence of Co(II) salts, for instance acetate. However, this reaction also has disadvantages that the best yields are achieved when working in expensive solvents (for instance N,N-dimethylacetamide) and in anhydrous conditions.

On the contrary, the catalytic oxidation of vicinal diols with aqueous $H_2O_2$ is of considerable industrial interest due to the relatively low cost of the oxidizer and to the absence of a reduction product to be disposed of. The oxidative scission of vicinal diols with $H_2O_2$ is, however, characterized by poor selectivity.

US-Patent No. US-A-3855257 discloses a process for preparing carboxylic acids comprising oxidizing an internal vicinal glycol with oxygen in the presence of a catalytic amount of a mixture comprising a cobalt (II) salt of an organic acid and a compound selected from peroxidized tungstic acid, peroxidized tungstic oxide, peroxidized molybdic acid and peroxidized molybdic oxide in a polar, aprotic solvent.

The present invention aims to provide a process for the preparation of monocarboxylic and bicarboxylic acids from vicinal diols soluble in water, by making use of aqueous $H_2O_2$, in which there is used an inexpensive catalytic system, and which ensures a high selectivity of the desired acid.

The present invention provides a process for the preparation of monocarboxylic and bicarboxylic acids by means of the oxidative scission of vicinal, terminal or non-terminal (internal), diols soluble in water and optionally containing functional groups inert under the reaction conditions, wherein a said vicinal diol is reacted in an aqueous solution with $H_2O_2$, at a temperature from 0 to 120°C and at a pH value from 0.5 to 4, in the presence of a catalytic system comprising tungstic acid or an alkali metal tungstate and, optionally, phosphoric or arsenic acid or an alkali metal salt thereof.

The reactions are as follows:

$R_1$-CHOH-CHOH-$R_2$+3$H_2O_2$ ⟶

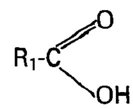 + 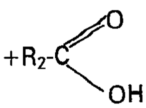 +4$H_2O$ (1)

$R_1$-CHOH-$CH_2$OH + 4 $H_2O_2$ ⟶

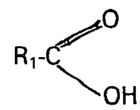 + $CO_2$ + 6 $H_2O$ (2)

where $R_1$ and $R_2$ are hydrocarbon groups (possibly containing functional groups that are inert under the reaction conditions), such as to allow the solubility in water of the vicinal diols.

As may be seen, reaction (1), concerning the non-terminal vicinal diols, produces two different carboxylic acids:

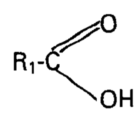 and 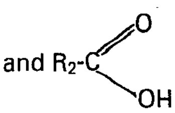

however, if $R_1$ = $R_2$ there is obtained one acid only.

When the vicinal diol $R_1$-CHOH-$CH_2$OH is a terminal diol, there is formed

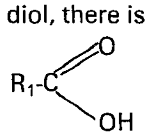

and formic acid which may be oxidized to $CO_2$ in the reaction medium; in such a case reaction (2) occurs.

If the non-terminal vicinal diol is a cyclic one, there is formed a bicarboxylic acid.

As already defined, the catalytic system always contains a first component, that is tungstic acid or an alkali metal tungstate and, optionally, a second

component selected from phosphoric acid or an alkali metal phosphate and arsenic acid or an alkali metal arsenate. Preferably, the catalytic system contains both components; the two-component system, in fact, ensures better yields.

The catalytic system may be formed in situ in the reaction medium, by introducing into the reactor, together with the vicinal diol, $H_2O_2$ and water, a compound of W and, optionally, of P or As capable of forming, in the reaction medium, a tungstate ion and, possibly, a phosphate or an arsenate ion.

Thus, there may be used as precursors of the first component of the catalytic system, i.e. of the tungstate ion, for example $WO_2$, $W_2O_5$, $WO_3$, $WS_2$, $WS_3$, $WCl_6$, $WOCl_4$ and $W(CO)_6$. There may be used as precursors of the phosphate ion or arsenate ion, for example $P_2O_5$, $As_2O_5$, $PCl_5$, $AsCl_5$, $POCl_3$, $AsOCl_3$ and polyphosphoric acid.

The starting vicinal diols may contain functional groups inert under reaction conditions. They may contain, for instance, from 1 to 3 such groups, either the same as or different from each other. Such functional groups, also chosen so as to allow the solubility of the vicinal diols in water, are for example Cl, F and COOH.

Water-soluble vicinal diols that may be conveniently used as starting substances are, for example: 1,2-cyclobutanediol, 1,2-cyclopentanediol, 1,2-cyclohexanediol, 1,2-cycloheptanediol, phenylethanediol, 1,2-propanediol, 1,2-butanediol, 2,3-butanediol, 1,2-pentanediol, 1,2-hexanediol, 3,4-hexanediol and 3-chloro-1,2-propanediol.

The reaction is usually conducted under vigorous stirring, after having dissolved in water the catalytic system, the hydrogen peroxide and the vicinal diol and, possibly, after having adjusted the pH of the resulting solution with dilute inorganic acid or base (e.g. HCl, $H_2SO_4$ or NaOH).

The pH value of the solution is in general from 0.5 to 4, but is preferably from about 2 to about 3.

The temperature is in general from 0 to 120 °C. Practically the temperature is determined on the basis of the reactivity of the diol and of the stability of the $H_2O_2$. Preferably the temperature is from about 60 to about 90 °C.

The reaction is usually conducted at substantially atmospheric pressure. The duration of the reaction depends on the reactivity of the vicinal diol used; in general, it takes from 5 to 15 hours to complete the reaction.

The catalytic system is preferably used in an amount of from 0.01 to 1 mol of W per mol of vicinal diol, more preferably in an amount of from 0.02 to 0.15 mols of W per mol of substrate.

When the catalytic system contains both components, these are used in a ratio, expressed as mols of W with respect to mols of P or As, of from 12 to 0.1, more preferably from about 4 to about 0.25.

The vicinal diol and the $H_2O_2$ are essentially used in molar ratios corresponding to the stoichiometry of reactions (1) and (2). However, there is preferably used a moderate excess of $H_2O_2$ (for instance 10%) with respect to the stoichiometry.

The concentration of the vicinal diol in the reaction mixture varies in general from 1 to 40% by weight, but is more preferably from about 15 to about 30%. The concentration of the $H_2O_2$ in the reaction mixture in general varies from 5 to 60% by weight, but is more preferably from about 15 to about 30% by weight.

At the end of the reaction, the acid or acids are recovered from the reaction medium utilizing conventional techniques.

The invention will be further described with reference to the following illustrative examples.

Example 1

Into a 100 ml flask, fitted with a reflux condenser and a magnetic stirrer, there were loaded 0.8 g of $Na_2WO_4 \cdot 2H_2O$ (about 2.4 mmols). 1.18 ml of $H_3PO_4$ at a concentration of 400 g/lt (about 4.8 mmols), 14,9 ml of $H_2O_2$ at a concentration of 400 g/lt (175.3 mmols), and 6 g of 1,2-cyclohexanediol (cis+trans, 51,7 mmols).

The pH of the resulting solution was then adjusted to value 2 by the addition of a few drops of 10% NaOH, whereupon the solution was brought to 70 °C, under vigorous stirring, and was then maintained at this temperature for 14.5 h.

At the end of this time, the mixture was allowed to rest overnight in a refrigerator (0–5 °C).

After filtering the crystals that had formed, through the solution there was first bubbled $SO_2$, until complete destruction of the residual $H_2O_2$ occurred, then $N_2$ in order to remove the excess of $SO_2$. The solution was then basified with NaOH at 10% concentration to obtain a pH value equal to 8, followed by drying at 60 °C under vacuum. The residue was extracted (during 40 min) with acetone at boiling temperature, and by subsequent evaporation of the acetonic solution obtained after filtering, there were recovered 60 mg of 1,2-cyclohexanediol.

The solid insoluble in acetone was, on the other hand, dissolved again in the minimum volume of water.

The solution was then acidified with a few drops of concentrated HCl and was then allowed to crystallize in a refrigerator. The crystals thus obtained, added to those already previously obtained, were washed with 1,2-dichloroethane, then with icy water (2 ml) and then first dried at a water pump, and then in an oven for two hours at 80 °C.

Thereby there were obtained 7.05 g of adipic acid with a 98.9% purity (titre determined by gas chromatography), which corresponded to a selectivity (calculated on the diol) in adipic acid of 93.3% and to a conversion of the cyclohexanediol equal to 99.0%.

Example 2

0.8 g of $Na_2WO_4 \cdot 2H_2O$ (about 2.4 mmols), 1.18 ml of $H_3PO_4$ at a concentration of 400 g/lt (about 4.8 mmols), 17 ml of $H_2O_2$ at a concentration of 400 g/lt (200 mmols) and 6 g of 1-phenyl-1,2-ethanediol (43.5 mmols) were loaded into a

100 ml flask, provided with a reflux condenser and a magnetic stirrer.

The pH value of the resulting solution was then adjusted to 2 by the addition of a few drops of 10% NaOH, whereupon the solution was brought to 70 °C, under vigorous stirring, for a period of 10 h. At the end of this time, after the addition of 30% NaOH until complete dissolution of the solid that had formed occurred (about 6 ml), the residual $H_2O_2$ was destroyed with $SO_2$.

If necessary, the pH value is adjusted again to a slightly alkaline pH with NaOH whereupon the solution was then dried under vacuum at 60 °C. The residue was extracted (for 40 min) with ether under reflux conditions and, by evaporation of the etheric solution obtained after filtering, there were recovered 320 mg of 1-phenyl-1,2-ethanediol. The solid unsoluble in ether was, on the other hand, dissolved again in the minimum volume of water and then acidified with 10% HCl.

Benzoic acid precipitated was filtered, dried at a water pump and dissolved again in ether. By evaporation, after filtering, of the etheric solution, there were obtained 4.07 g of benzoic acid at a 98.8% purity, which corresponded to a selectivity in benzoic acid of 80.1% and to a conversion of the 1-phenyl-1,2-ethanediol equal to 94.7%.

Example 3

Into a 50 ml flask, provided with a reflux condenser and a magnetic stirrer, there were loaded 0.8 g of $Na_2WO_4 \cdot 2H_2O$ (about 2.4 mmols), 1.5 g of $Na_2HAsO_4 \cdot 7H_2O$ (4.8 mmols), 1.5 cc of $H_2O$, 7.5 cc of $H_2O_2$ at a concentration of 400 g/lt (88.2 mmols) and 2.76 g of 1-phenyl-1,2-ethanediol (20 mmols).

By the addition of $H_2SO_4$ at a 30% concentration the resulting solution was adjusted to a pH value of 2 and was then brought to 70 °C, under vigorous stirring, for 5 h.

Thereupon the procedure described in example 2 was followed. There were recovered 120 mg of 1-phenyl-1,2-ethanediol and there were obtained 1.62 g of benzoic acid at a 99.3% purity, which corresponded to a selectivity in benzoic acid of 68.9% and to a conversion of the 1-phenyl-1,2-ethanediol equal to 95.7%.

Example 4

Into a 50 ml flask provided with a reflux condenser and a magnetic stirrer, there were loaded 0.8 g of finely ground $Na_2WO_4 \cdot 2H_2O$ (about 2.4 mmols), 1.2 ml of water, 7.2 ml of hydrogen peroxide in a 400 g/lt concentration (84.7 mmols) and 3 g of 1,2-cyclohexanediol (cis+trans, about 25.9 mmols).

By the addition of HCl the resulting solution was adjusted to a pH of 2, whereafter it was brought to 70 °C, under vigorous stirring, for 7 h. Thereupon the procedure described in example 1 was followed.

There were recovered 40 mg of 1,2-cyclohexanediol while there were obtained 2.96 g of adipic acid at a 98.4% purity, which corresponded to a selectivity in adipic acid of 78.2% and to a conversion of the cyclohexanediol equal to 98.7%.

## Claims

1. A process for preparing monocarboxylic and bicarboxylic acids by means of oxidative scission of vicinal diols with $H_2O_2$, characterized by reacting, in an aqueous solution, a vicinal diol, either terminal or non-terminal, which is soluble in water and which optionally contains one or more functional groups that are inert under the reaction conditions, with $H_2O_2$ at a temperature from 0 to 120 °C and at a pH value from 0.5 to 4, in the presence of a catalytic system comprising tungstic acid or an alkali metal tungstate and optionally phosphoric or arsenic acid or an alkali metal salt thereof.

2. A process as claimed in claim 1, characterized in that the catalytic system contains both tungstic acid or an alkali metal tungstate and phosphoric or arsenic acid or an alkali metal salt thereof.

3. A process as claimed in claim 2, characterized in that the molar ratio

$$\frac{W}{P \text{ or } As}$$

is from 12 to 0.1.

4. A process as claimed in any of claims 1 to 3, characterized in that the catalytic system is prepared in situ in the reaction medium, by introducing into the reaction medium a tungsten compound and, optionally, a phosphorous or arsenic compound, capable of forming, in the said medium, a tungstate ion and a phosphate or arsenate ion.

5. A process as claimed in claim 4, characterized in that the tungsten compound capable of forming a tungstate ion is selected from $WO_2$, $W_2O_5$, $WO_3$, $WS_2$, $WS_3$, $WCl_6$, $WOCl_4$ and $W(CO)_6$.

6. A process as claimed in claim 4 or 5, characterized in that the phosphorus or arsenic compound capable of forming a phosphate or arsenate ion is selected from $P_2O_5$, $As_2O_5$, $PCl_5$, $AsCl_5$, $POCl_3$, $AsOCl_3$ and polyphosphoric acid.

7. A process as claimed in any of claims from 1 to 6, characterized in that the reaction is conducted at a pH value of from about 2 to about 3.

8. A process as claimed in any of claims 1 to 7, characterized in that the reaction is conducted at a temperature of from about 60 to about 90 °C.

9. A process as claimed in any of claims 1 to 8, characterized in that the catalytic system is used in an amount of from 0.01 to 1 mol of W per mol of vicinal diol.

## Patentansprüche

1. Ein Verfahren zur Herstellung von Monocarbon- oder Dicarbonsäuren durch oxidative Spaltung von vicinalen Diolen mit $H_2O_2$, dadurch gekennzeichnet, dass man in einer wässrigen Lösung ein endständiges oder nicht-endständiges vicinales Diol, das in Wasser löslich ist, und gegebenenfalls eine oder mehrere funktionelle Gruppen, die unter den Reaktionsbedingungen inert

sind, enthält, mit $H_2O_2$ bei einer Temperatur von 0 bis 120 °C und bei einem pH-Wert von 0,5 bis 4 in Anwesenheit eines katalytischen Systems, das Wolframsäure oder ein Alkalimetallwolframat und gegebenenfalls Phosphor- oder Arsensäure oder ein Alkalimetallsalz derselben umfasst, umsetzt.

2. Verfahren nach Anspruch 1, dadurch gekennzeichnet, dass das Katalysatorsytem sowohl Wolframsäure oder ein Alkalimetallwolframat als auch Phosphor- oder Arsensäure oder ein Alkalimetallsalz derselben enthält.

3. Verfahren nach Anspruch 2, dadurch gekennzeichnet, dass das molare Verhältnis

$$\frac{W}{P \text{ or } As}$$

von 12 bis 0,1 beträgt.

4. Verfahren nach irgendeinem der Ansprüche 1 bis 3, dadurch gekennzeichnet, dass das Katalysatorsystem in situ im Reaktionsmedium hergestellt wird, indem man in das Reaktionsmedium eine Wolframverbindung und, gegebenenfalls eine Phosphor- oder Arsenverbindung, die in diesem Medium ein Wolframation oder ein Phosphat- oder Arsenation bilden können, einführt.

5. Verfahren nach Anspruch 4, dadurch gekennzeichnet, dass die zur Bildung eines Wolframations fähige Wolframverbindung ausgewählt ist aus $WO_2$, $W_2O_5$, $WO_3$, $WS_2$, $WS_3$, $WCl_6$, $WOCl_4$ und $W(CO)_6$.

6. Verfahren nach Anspruch 4 oder 5, dadurch gekennzeichnet, dass die zur Bildung eines Phosphat- oder Arsenations fähige Phosphor- oder Arsenverbindung ausgewählt ist aus $P_2O_5$, $As_2O_5$, $PCl_5$, $AsCl_5$, $POCl_3$, $AsOCl_3$ und Polyphosphorsäure.

7. Verfahren nach irgendeinem der Ansprüche 1 bis 6, dadurch gekennzeichnet, dass die Reaktion bei einem pH-Wert von etwa 2 bis etwa 3 durchgeführt wird.

8. Verfahren nach irgendeinem der Ansprüche 1 bis 7, dadurch gekennzeichnet, dass die Reaktion bei einer Temperatur von etwa 60 bis etwa 90 °C durchgeführt wird.

9. Verfahren nach irgendeinem der Ansprüche 1 bis 8, dadurch gekennzeichnet, dass das Katalysatorsystem in einer Menge von 0,01 bis 1 Mol W pro Mol vicinales Diol verwendet wird.

**Revendications**

1. Un procédé de préparation des acides monocarboxyliques et dicarboxyliques au moyen d'une coupure oxydante de diols vicinaux par $H_2O_2$, caractérisé par la réaction, en milieu aqueux, d'un diol vicinal terminal ou non terminal, soluble dans l'eau et qui contient éventuellement un ou plusieurs groupes fonctionnels inertes dans les conditions de la réaction, avec $H_2O_2$ à une température comprise entre 0 et 120 °C et un pH comprise entre 0,5 et 4, en présence d'un système catalytique comprenant l'acide tungstique ou un tungstate de métal alcalin et, éventuellement, l'acide phosphorique ou l'acide arsenique ou un de leur métal alcalin.

2. Un procédé selon la revendication 1, caractérisé en ce que le système catalytique contient à la fois l'acide tungstique ou un tungstate de métal alcalin et l'acide phosphorique ou l'acide arsenique ou leur sel de métal alcalin.

3. Un procédé selon la revendication 2, caractérisé en ce que le rappaort molaire W/P ou As est compris entre 12 et 0,1.

4. Un procédé selon l'une quelconque des revendications 1 à 3, caractérisé en ce que le système catalytique est préparé in situ dans le milieu réactionnel par introduction dans le milieu réactionnel d'un composé de tungstène et, éventuellement d'un composé de phosphore ou d'arsenic, capable de former dans ledit milieu un ion tungstate et un ion phosphate ou arséniate.

5. Un procédé selon la revendication 4, caractérisé en ce que le composé de tungstène capable de former un ion tungstate est choisi dans la liste comprenant $WO_2$, $W_2O_5$, $WO_3$, $WS_2$, $WS_3$, $WCl_6$, $WOCl_4$ et $W(CO)_6$.

6. Un procédé selon l'une quelconques des revendications 4 ou 5, caractérisé en ce que le composé de phosphore ou d'arsenic capable de former un ion phosphate ou arséniate est choisi dans la liste comprenant $P_2O_5$, $As_2O_5$, $PCl_5$, $AsCl_5$, $POCl_3$, $AsOCl_3$ et l'acide polyphosphorique.

7. Un procédé selon l'une quelconque des revendications 1 à 6, caractérisé en ce que la réaction est effectuée à un pH compris entre environ 2 et environ 3.

8. Un procédé selon l'une quelconque des revendications 1 à 7, caractérisé en ce que la réaction est effectuée à une température comprise entre environ 60 et environ 90 °C.

9. Un procédé selon l'une quelconque des revendications 1 à 8, caractérisé en ce que le système catalytique est utilisé à raison de 0,01 à 1 mole de W par mole de diol vicinal.